# EUROPEAN PATENT APPLICATION

(11) **EP 4 438 587 A1**
(43) Date of publication of application: **02.10.2024**
(21) Application number: 22906497.7
(22) Date of filing: 12.12.2022
(51) Int. Cl.: C07C 67/08, C07C 67/24, C07C 67/31, C07C 69/675, C07C 67/37, C07C 69/708, B01J 29/70, B01J 29/08, B01J 29/40, B01J 29/65

(54) **METHOD FOR PREPARING METHYL ESTER COMPOUND**

(30) Priority: 13.12.2021 CN 202111522952
(71) Applicant: Dalian Institute Of Chemical Physics, Chinese Academy Of Sciences, Liaoning 116023 (CN)
(72) Inventor: NI, Youming, Dalian, Liaoning 116023 (CN); ZHU, Wenliang, Dalian, Liaoning 116023 (CN); LIU, Zhongmin, Dalian, Liaoning 116023 (CN)
(74) Representative: Bayramoglu et al.
(86) International application number: PCT/CN2022/138385
(87) International publication number: WO 2023/109752

(57) **Abstract**

A method for preparing a methyl ester compound, which comprises: subjecting a raw material of a carboxyl compound, methanol and/or dimethyl ether to an esterification reaction to obtain the methyl ester compound, where the general formula I of the carboxyl compound is R-CH₂-COOH, where R is a methoxy or hydroxyl; and the methyl ester compound comprises at least one of methyl methoxyacetate and methyl glycolate. This application also discloses a method for preparing a methyl glycolate, comprising the steps of: a) subjecting methylal and carbon monoxide to a carbonylation reaction to obtain methyl methoxyacetate; b) subjecting the methyl methoxyacetate and water to a hydrolysis reaction to obtain the methyl glycolate; and c) subjecting glycolic acid, methoxyacetic acid, methanol, and dimethyl ether to an esterification reaction to obtain the methyl glycolate. The method can greatly simplify the separation process, save the energy consumption, and provides a new synthesis route for synthesizing a methyl ester compound, especially methyl glycolate.

## Description

### TECHNICAL FIELD

The present application relates to a method for preparing a methyl ester compound, which belongs to the field of catalytic chemistry.

### BACKGROUND

Methyl glycolate (HOCH₂COOCH₃, MG) is an important platform compound for producing ethylene glycol through hydrogenation, producing glycolic acid through hydrolysis, and producing polyglycolic acid (PGA) through polymerization. Ethylene glycol is the monomer for the widely used polyethylene terephthalate (PET) material, which is in high demand in the market. Glycolic acid is an excellent chemical detergents and cosmetic raw material, and is also capable of polymerizing itself to generate PGA. On January 1, 2021, the strictest plastic restriction order in history officially came into effect in China. Disposable non-biodegradable plastic straws, non-biodegradable plastic packaging, etc. have been explicitly banned, the switch to green materials has been the general trend, and the degradable plastics industry has become a hot spot of market concern recently. Polyglycolic acid PGA is the simplest linear polyester among polyhydroxyalkanoates, which is a fully biodegradable material and can be synthesized by condensation of monomers such as methyl glycolate and glycolic acid. Its degradation conditions are mild, and under the action of water and microorganisms, it can realize rapid degradation in the natural environment, and the final degradation products are carbon dioxide and water. In addition, PGA can be degraded in seawater, and its degradation products are harmless to human body and the environment.

Methyl glycolate can be prepared by formaldehyde carbonylation method. Although the raw material is cheap and easy to obtain, it needs to be carried out under the conditions of high temperature, high pressure, strong liquid acid, and organic solvent; the equipment is easy to be corroded, and the product is difficult to be purified, which leads to the high cost of industrial production. In recent years, with the large-scale industrial application of "coal to ethylene glycol" technology, the method of partial hydrogenation of its intermediate product dimethyl oxalate to methyl glycolate has been widely concerned. However, on the one hand, the catalyst for the partial hydrogenation of dimethyl oxalate is still immature, with low conversion efficiency and poor stability; on the other hand, the production process of dimethyl oxalate is long and costly; this seriously restricts the development of this method. At present, the industrial production technology of methyl glycolate monomer is still immature, which leads to insufficient production capacity and expensive price of PGA plastic, limiting its large-scale substitution application.

### SUMMARY

To overcome the defects in the prior art, the present application provides a green, economical, and efficient technical route for synthesizing methyl esters, especially methyl glycolate.

According to a first aspect of the present application, there is provided a method for preparing a methyl ester compound, where a raw material containing methoxyacetic acid, methanol and/or dimethyl ether is passed through a reactor and an esterification reaction occurs to obtain a product containing methyl methoxyacetate. Through the easily accessible esterification reaction of methoxyacetic acid and glycolic acid, not only the yield of methyl glycolate can be increased, but also the separation of methoxyacetic acid and glycolic acid is not required; this can substantially simplify the separation process and save energy consumption.

As a specific embodiment, the method of esterification of methoxyacetic acid and glycolic acid in this application comprises passing a raw material containing methoxyacetic acid, glycolic acid, methanol, and dimethyl ether through a reactor to undergo an esterification reaction to produce methyl methoxyacetate and methyl glycolate under a predetermined reaction condition.

According to the mechanism of hydrolysis reaction, the hydrolysis reaction of methyl methoxyacetate can generate other hydrolysis products such as methoxyacetic acid, glycolic acid, methanol, and dimethyl ether, in addition to the target product methyl glycolate. Due to the small use of methoxyacetic acid and the high energy consumption for purification of low concentration glycolic acid, it is possible to further generate methyl methoxyacetate and methyl glycolate through an easily carried out esterification reaction. In this way, it can not only increase the yield of methyl glycolate, but also greatly simplify the separation process and save energy consumption.

The esterification reaction mainly includes the following reactions:

CH₃OCH₂COOH + CH₃OH = CH₃OCH₂COOCH₃ + H₂O

HOCH₂COOH + CH₃OH = HOCH₂COOCH₃

CH₃OCH₂COOH + CH₃OCH₃ = CH₃OCH₂COOCH₃ + CH₃OH

HOCH₂COOH + CH₃OCH₃ = HOCH₂COOCH₃ + CH₃OH

The esterification method comprises passing a raw material containing a carboxyl compound, methanol, and/or dimethyl ether through a reactor to undergo an esterification reaction to obtain the methyl ester compound;
wherein the carboxyl compound is at least one selected from the group consisting of the compounds shown in Formula I;

R-CH₂-COOH Formula I

R is methoxy or hydroxy;
the methyl ester compound comprises at least one of methyl methoxyacetate, methyl glycolate.

Optionally, conditions of the esterification reaction are as follows: a reaction temperature ranges from 40°C to 300°C and a reaction pressure ranges from 0.1 MPa to 0.5 MPa.

The esterification reaction is carried out without a catalyst or under a condition of an esterification catalyst.

The esterification catalyst is a solid catalyst insoluble in the raw material and the product.

Optionally, the solid catalyst is at least one of an acidic molecular sieve, an acidic cation exchange resin.

Optionally, the esterification reaction is carried out on at least one inert composition selected from the group consisting of quartz sand, alumina, silicon oxide, silicon carbide, glass, ceramics.

As a specific embodiment, the reactor does not carry any catalyst or carries a solid catalyst insoluble in the raw material and the product.

Optionally, the esterification reaction of methoxyacetic acid and glycolic acid, where the methoxyacetic acid and glycolic acid themselves can act as catalysts, is an autocatalytic reaction, and thus can be performed without a catalyst.

Optionally, the solid catalyst insoluble in the raw material and the product includes an acidic solid catalyst and a non-acidic solid catalyst. The non-acidic solid catalyst mainly plays a role of promoting material mixing and dispersion; the acidic solid catalyst not only has the above effect, but also catalyzes the esterification reaction.

Optionally, the solid catalyst insoluble in the raw material and the product is spherical or cylindrical.

Optionally, the raw material is methoxyacetic acid, methanol and/or dimethyl ether; or
the raw material is glycolic acid, methanol and/or dimethyl ether; or
the raw material is methoxyacetic acid, glycolic acid, and methanol; or
the raw material is methoxyacetic acid, glycolic acid, and dimethyl ether; or
the raw material is methoxyacetic acid, glycolic acid, methanol, and dimethyl ether.

Optionally, the product further comprises methyl glycolate.

As a specific embodiment, the molar ratio of methoxyacetic acid, glycolic acid, methanol, and dimethyl ether in the raw material are arbitrary.

Optionally, the raw material contains methoxyacetic acid, glycolic acid, methanol, and dimethyl ether;
the molar ratio of methoxyacetic acid/glycolic acid in the raw material is from 3:1 to 10:1, and the molar ratio of (methanol + dimethyl ether)/ (methoxyacetic acid + glycolic acid) is from 1:1 to 5:1.

Optionally, the raw material does not contain methanol, the molar ratio of methoxyacetic acid/ glycolic acid is from 3:1 to 10:1, and the molar ratio of dimethyl ether/ (methoxyacetic acid + glycolic acid) is from 1:1 to 5:1.

Optionally, the raw material does not contain methanol and glycolic acid, and the molar ratio of dimethyl ether/methoxyacetic acid is from 1:1 to 5:1.

Optionally, the raw material does not contain dimethyl ether;
the molar ratio of methoxyacetic acid/glycolic acid is from 3:1 to 10:1, and the molar ratio of methanol/ (methoxyacetic acid + glycolic acid) is from 1:1 to 5:1.

Optionally, the reaction temperature may be selected from any value or a range value determined by any two of 40°C, 50°C, 60°C, 70°C, 80°C, 90°C, 100°C, 110°C, 120°C, 130°C, 140°C, 150°C, 160°C, 170°C, 180°C, 190°C, 200°C, 210°C, 220°C, 230°C, 240°C, 250°C, 260°C, 270°C, 280°C, 290°C, and 300°C.

Optionally, the reaction pressure may be selected from any value or a range value determined by any two of 0.1 MPa, 0.15 MPa, 0.20 MPa, 0.25 MPa, 0.30 MPa, 0.35 MPa, 0.40 MPa, 0.45 MPa, and 0.50 MPa.

Optionally, the molar ratio of methoxyacetic acid/glycolic acid may be selected from any value or a range value determined by any two of 3:1, 3.5:1, 4:1, 4.5:1, 5:1, 5.5:1, 6:1, 6.5:1, 7:1, 7.5:1, 8:1, 8.5:1, 9:1, 9.5:1, and 10:1.

Optionally, the molar ratio of (methanol + dimethyl ether)/ (methoxyacetic acid + glycolic acid) is any value or a range value determined by any two of 0.5:1, 1.0:1, 1.5:1, 2.0:1, 2.5:1, 3.0:1, 3.5:1, 4.0:1, 4.5:1, 5.0:1, 5.5:1, 6.0:1, 6.5:1, 7.0:1, 7.5:1, and 8.0:1.

Optionally, the raw material does not contain methanol or does not contain dimethyl ether, and the molar ratio of methoxyacetic acid/glycolic acid may be selected from any value or a range value determined by any two of 3:1, 3.5:1, 4:1, 4.5:1, 5:1, 5.5:1, 6:1, 6.5:1, 7:1, 7.5:1, 8:1, 8.5:1, 9:1, 9.5:1, and 10:1.

Optionally, the molar ratio of dimethyl ether (or methanol)/ (methoxyacetic acid + glycolic acid) is any value or a range value determined by any two of 1:1, 1.5:1, 2.0:1, 2.5:1, 3.0:1, 3.5:1, 4.0:1, 4.5:1, and 5.0:1.

Optionally, the raw material is generated through a hydrolysis reaction of methyl methoxyacetate.

Optionally, the raw material containing methoxyacetic acid, glycolic acid, methanol, and dimethyl ether is generated through a hydrolysis reaction of methyl methoxyacetate.

Optionally, the reactor is one of a fixed bed reactor or a kettle reactor.

The reactor is preferably a fixed bed reactor.

As a specific embodiment, the reaction process includes a carrier gas, and the carrier gas comprises at least one of nitrogen, argon, helium, hydrogen, carbon monoxide, carbon dioxide.

According to a second aspect of the present application, there is provided a method for preparing a methyl glycolate, where the method comprises the following steps:
a) passing a raw material containing methylal and carbon monoxide through a reactor carrying an acidic molecular sieve catalyst, and performing a carbonylation reaction under a predetermined condition to generate a product containing methyl methoxyacetate, dimethyl ether, and methyl formate, which is separated to obtain a methyl methoxyacetate;
b) passing a raw material containing the methyl methoxyacetate obtained in step a) and water through a reactor carrying an acidic molecular sieve catalyst, and performing a hydrolysis reaction under a predetermined condition to generate a product containing methyl glycolate, glycolic acid, methoxyacetic acid, methanol, and dimethyl ether, which is separated to obtain the methyl glycolate; and
c) passing the glycolic acid, the methoxyacetic acid, the methanol and dimethyl ether obtained in step b) through a reactor, and performing an esterification reaction under a predetermined condition to generate a product containing methyl methoxyacetate and methyl glycolate, which is separated to obtain the methyl glycolate.

The technical route composed of combination of steps a) to c) provides an industrially available route for the synthesis of methyl glycolate.

Where, the carbonylation reaction of methylal in step a) mainly includes the following reactions:

CH₃OCH₂OCH₃ + CO = CH₃OCH₂COOCH₃ (1)

2 CH₃OCH₂OCH₃ = 2 CH₃OCH₃ + HCOOCH₃ (2)

The hydrolysis reaction of methyl methoxyacetate in step b) mainly includes the following reactions:

CH₃OCH₂COOCH₃ + H₂O = HOCH₂COOCH₃ + CH₃OH (3)

CH₃OCH₂COOCH₃ + H₂O = CH₃OCH₂COOH + CH₃OH (4)

CH₃OCH₂COOCH₃ + 2 H₂O = HOCH₂COOH + 2 CH₃OH (5)

2 CH₃OH = CH₃OCH₃ + H₂O (6)

The esterification reaction in step c) mainly includes the following reactions:

CH₃OCH₂COOH + CH₃OH = CH₃OCH₂COOCH₃ + H₂O (7)

HOCH₂COOH + CH₃OH = HOCH₂COOCH₃ (8)

CH₃OCH₂COOH + CH₃OCH₃ = CH₃OCH₂COOCH₃ + CH₃OH (9)

HOCH₂COOH + CH₃OCH₃ = HOCH₂COOCH₃ + CH₃OH (10)

The reactions (3) to (10) above are reversible.

The dimethyl ether and methanol obtained through separation systems of a) and b) can be used for the preparation of methylal. The reactions involved are:

2 CH₃OH + O₂ = 2 HCHO +2 H₂O (11)

CH₃OCH₃ + O₂ = 2 HCHO + H₂O (12)

2 CH₃OH + HCHO = CH₃OCH₂OCH₃ + H₂O (13)

Synthesizing reactions (1)-(13) above, by combining reactions such as synthesis of methylal, carbonylation of methylal, hydrolysis of methyl methoxyacetate, and esterification of methoxyacetic acid and glycolic acid, the methyl glycolate can be synthesized using methanol, carbon monoxide, and oxygen, with the following overall reaction equation:

4 CH₃OH + O₂+2CO = 2 HOCH₂COOCH₃ + 2 H₂O (14)

In summary, it can be seen that the technical route composed of the combination of steps a) to c) above is therefore able to achieve full utilization of the non-target products, with only methyl formate (HCOOCH₃), which has a very low selectivity, as a by-product.

Optionally, the method further comprises step a'): returning the unreacted methylal and carbon monoxide in step a) to the reactor of step a) to continue the carbonylation reaction.

Optionally, the method further comprises step b'): returning the unreacted methyl methoxyacetate in step b) to the reactor of step b) to continue the hydrolysis reaction with water.

Optionally, the method further comprises step c'): passing the product methyl methoxyacetate obtained in step c) into the reactor of step b) for the hydrolysis reaction with water.

Optionally, the acidic molecular sieve catalyst in step a) or step b) is at least one selected from the group consisting of an acidic molecular sieve with MFI structure, an acidic molecular sieve with Y structure, an acidic molecular sieve with FER structure, an acidic molecular sieve with BEA structure, an acidic molecular sieve with MOR structure, an acidic molecular sieve with MWW structure.

Optionally, the acidic molecular sieve catalyst in step a) or step b) is at least one of a hydrogen-type ZSM-5 molecular sieve, a hydrogen-type Y molecular sieve, a hydrogen-type ZSM-35 molecular sieve, a hydrogen-type β molecular sieve, a hydrogen-type mordenite molecular sieve, and a hydrogen-type MCM-22 molecular sieve.

Optionally, step a) or step b) further comprises introducing a carrier gas into the reactor, and the carrier gas comprises at least one of nitrogen, argon, helium, hydrogen, carbon monoxide, or carbon dioxide in any amount.

Optionally, reaction conditions in step a) are: a reaction temperature ranges from 60°C to 140°C, a reaction pressure ranges from 2 MPa to 10 MPa, a weight hourly space velocity of the methylal ranges from 0.2 h⁻¹ to 10.0 h⁻¹, and a molar ratio of the carbon monoxide to the methylal ranges from 2: 1 to 20:1.

Optionally, the reaction temperature in step a) is any value or a range value determined by any two of 60°C, 65°C, 70°C, 75°C, 80°C, 85°C, 90°C, 100°C, 110°C, 120°C, 130°C, and 140°C.

Optionally, the reaction pressure in step a) is any value or a range value determined by any two of 2 MPa, 2.5 MPa, 3.0 MPa, 3.5 MPa, 4.0 MPa, 4.5 MPa, 5.0 MPa, 5.5 MPa, 6.0 MPa, 6.5 MPa, 7.0 MPa, 7.5 MPa, 8.0 MPa, 8.5 MPa, 9.0 MPa, 9.5 MPa, and 10.0 MPa.

Optionally, the weight hourly space velocity of the methylal in step a) is any value or a range value determined by any two of 0.2 h⁻¹, 0.5 h⁻¹, 0.8 h⁻¹, 1.0 h⁻¹, 1.5h⁻¹, 2.0 h⁻¹, 2.5 h⁻¹, 3.0 h⁻¹, 3.5 h⁻¹, 4.0 h⁻¹, 4.5 h⁻¹, 5.0 h⁻¹, 5.5 h⁻¹, 6.0 h⁻¹, 6.5 h⁻¹, 7.0 h⁻¹, 7.5 h⁻¹, 8.0 h⁻¹, 8.5 h⁻¹, 9.0 h⁻¹, 9.5 h⁻¹, and 10.0 h⁻¹.

Optionally, the molar ratio of the carbon monoxide to the methylal in step a) is any value or a range value determined by any two of 2:1, 3:1, 4:1, 5:1, 6:1, 7:1, 8:1, 9:1, 10:1, 11:1, 12:1, 13:1, 14:1, 15:1, 16:1, 17:1, 18:1, 19:1, and 20:1.

Optionally, reaction conditions in step b) are: a reaction temperature ranges from 140°C to 220°C, a reaction pressure ranges from 0.1 MPa to 0.5 MPa, a weight hourly space velocity of the methyl methoxyacetate ranges from 0.1 h⁻¹ to 3.0 h⁻¹, and a molar ratio of the water to the methyl methoxyacetate ranges from 0.5:1 to 8:1.

Optionally, reaction conditions in step c) are: a reaction temperature ranges from 40°C to 300°C, a reaction pressure ranges from 0.1 MPa to 0.5 MPa, and a molar ratio of (methanol + dimethyl ether)/ (glycolic acid+ methoxyacetic acid) ranges from 0.5: 1 to 8:1.

Optionally, a reaction state in step a) is a gas-liquid-solid three-phase reaction state.

Optionally, the esterification reaction in step c) is carried out without a catalyst or under an esterification catalyst condition.

Optionally, the esterification catalyst is a solid catalyst insoluble in the raw material and the product.

Optionally, the solid catalyst is at least one of an acidic molecular sieve, an acidic cation exchange resin;

Optionally, the esterification reaction is carried out on at least one inert composition selected from the group consisting of quartz sand, alumina, silicon oxide, silicon carbide, glass, ceramics.

Preferably, the esterification reaction is carried out on quartz sand.

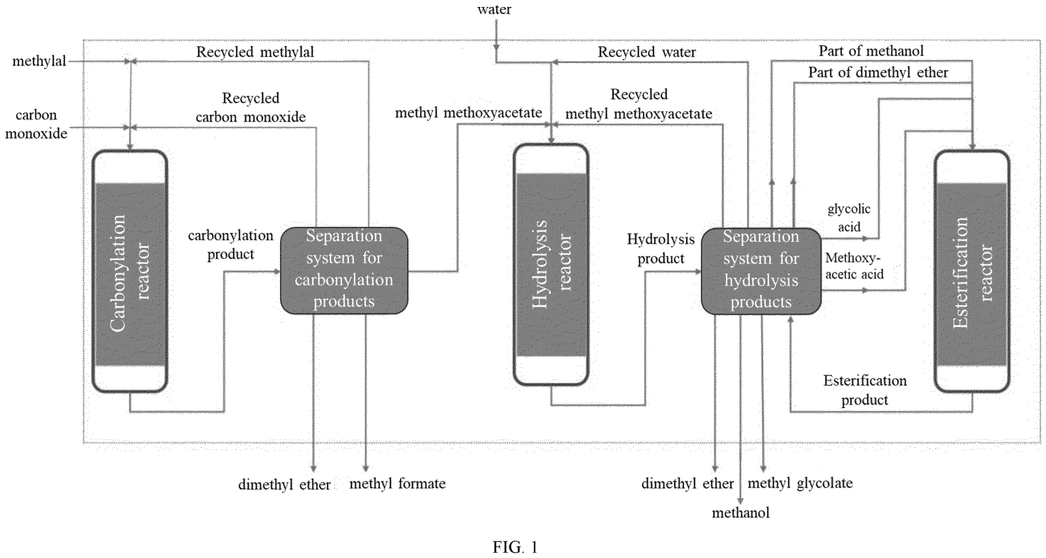

The core part of the technical route involved in the present invention is shown in Figure 1. The reactor for the carbonylation reaction is loaded with an acidic molecular sieve catalyst, and the hydrolysis reactor for the hydrolysis reaction is loaded with an acidic molecular sieve catalyst. The esterification reaction is autocatalytic, and in order to enhance mass transfer, the esterification reactor may be loaded with inert quartz sand particles.

Optionally, the carbonylation reactor contains a single fixed bed reactor or may contain multiple fixed bed reactors connected in parallel or in series. In order to facilitate the removal of the reaction heat, the carbonylation reactor generally chose a multitubular fixed bed reactor.

Optionally, the hydrolysis reactor contains a single fixed bed reactor or may contain multiple fixed bed reactors connected in parallel or in series. The hydrolysis reactors generally choose an adiabatic fixed bed reactor.

Separation system for separating carbonylation products contains commonly used chemical equipment in chemical separation units such as gas-liquid separation tanks and distillation towers.

Separation system for separating hydrolysis products also contains commonly used chemical equipment in chemical separation units such as gas-liquid separation tanks and distillation towers.

Since the types of hydrolysis products already include all esterification products, one separation system can be shared.

Beneficial effects that can be produced by the present application include:
(1) The method described in this application is simple, efficient, and easy to perform.
(2) Through the esterification reaction of methoxyacetic acid and glycolic acid which is easily carried out, not only the yield of methyl glycolate can be increased, but also there is no need to separate methoxyacetic acid and glycolic acid; this can substantially simplify the separation process and save energy consumption.
(3) The present application provides a completely new synthesis route method for methyl glycolate, with mild reaction conditions, low by-product selectivity, high selectivity of the target product methyl glycolate, and high atomic economy; the reaction and separation equipment used in the route are conventional equipment, which facilitates single-set large-scale production; no raw materials or catalysts containing sulfur, nitrogen, chlorine, and other elements are introduced into the route, and the product methyl glycolate is of high quality; the catalysts used in this route are acidic molecular sieve catalysts, and no expensive noble metal catalysts are needed.

### BRIEF DESCRIPTION OF THE DRAWINGS

FIG. 1 shows a flowchart of the synthesis route for methyl glycolate of the present application.

### DETAILED DESCRIPTION OF THE EMBODIMENTS

The present application will be described in detail below with reference to embodiments, but the present disclosure is not limited to these embodiments.

Endpoints and any values of ranges disclosed in the present application are not limited to the exact ranges or values, and these ranges or values should be understood as including approximate ranges or values. For numeric ranges, endpoint values and individual point values of each range may be combined with each other to obtain one or more new numeric ranges, and these numeric ranges should be considered to be specifically disclosed herein.

The present application will be described in detail below with reference to embodiments, but the present disclosure is not limited to these embodiments.

Unless otherwise specified, raw materials and catalyst used in the examples of the present application are purchased by commercial ways.

The key technology involved in the present invention are the three reactions of carbonylation, hydrolysis and esterification shown in FIG. 1, and they are therefore illustrated in separate embodiments.
1)The method of analysis for the carbonylation reaction of methylal is as follows:
   Products and unreacted raw material were analyzed using an Agilent 7890A gas chromatograph with its FID detector connected to a DB-FFAP capillary column and its TCD detector connected to a Porapak Q packed column. The reaction product passes through a backpressure valve and is heated to a vaporized state for on-line analysis by chromatography. Both conversion rate and selectivity were calculated based on the carbon moles of methylal: Methylal conversion rate = [(Carbon moles of methylal in raw material) - (Carbon moles of methylal in product)] ÷ (Carbon moles of methylal in raw material) × (100%) Methyl methoxyacetate selectivity = (Carbon moles of methyl methoxyacetate after removal of carbonyl groups in product) ÷ [(Carbon moles of methylal in raw material) - (Carbon moles of methylal in product)] × (100%) Dimethyl ether selectivity = (Carbon moles of dimethyl ether in product) ÷ [(Carbon moles of methylal in raw material) - (Carbon moles of methylal in product)] × (100%) Methyl formate selectivity = (Carbon moles of methyl formate in product) ÷ [(Carbon moles of methylal in raw material) - (Carbon moles of methylal in product)] × (100%)
2) The method of analysis for the hydrolysis reaction of methyl methoxyacetate is as follows:
   Products other than glycolic acid and unreacted raw material were analyzed using an Agilent 7890B gas chromatograph with its FID detector connected to a DB-FFAP capillary column and its TCD detector connected to a Porapak Q packed column. The glycolic acid was analyzed using a liquid chromatograph with a C18 column for separation and a UV detector. Conversion rate and selectivity were calculated based on carbon moles: Methyl methoxyacetate conversion rate = [(Carbon moles of methyl methoxyacetate in the feed) - (Carbon moles of methyl methoxyacetate in the discharge)] ÷ (Carbon moles of methyl methoxyacetate in the feed) × 100% Methyl glycolate selectivity = [(Carbon moles of methyl glycolate in the discharge)] ÷ [(Carbon moles of methyl methoxyacetate in the feed) - (Carbon moles of methyl methoxyacetate in the discharge)] × 100% Methoxyacetic acid selectivtiy = [(Carbon moles of methoxyacetic acid in the discharge)] ÷ [(Carbon moles of methyl methoxyacetate in the feed) - (Carbon moles of methyl methoxyacetate in the discharge)] × 100%.Glycolic acid selectivity = [(Carbon moles of glycolic acid in the discharge)] ÷ [(Carbon moles of methyl methoxyacetate in the feed) - (Carbon moles of methyl methoxyacetate in the discharge)] × 100%. Methanol selectivity = [(Carbon moles of methanol in the discharge)] ÷ [(Carbon moles of methyl methoxyacetate in the feed) - (Carbon moles of methyl methoxyacetate in the discharge)] × 100%. Dimethyl ether selectivity = [(Carbon moles of dimethyl ether in the discharge)] ÷ [(Carbon moles of methyl methoxyacetate in the feed) - (Carbon moles of methyl methoxyacetate in the discharge)] × 100%.
3) The method of analysis for the esterification reaction is as follows:
   Products other than glycolic acid and unreacted raw material were analyzed using an Agilent 7890B gas chromatograph with its FID detector connected to a DB-FFAP capillary column and its TCD detector connected to a Porapak Q packed column. The glycolic acid was analyzed using a liquid chromatograph with a C18 column for separation and a UV detector. Considering the directed esterification of methoxyacetic acid to methyl methoxyacetate and glycolic acid to methyl glycolate, the results of the esterification reaction were calculated only for the conversion rate of methoxyacetic acid and glycolic acid. Conversion rate of glycolic acid = [(Moles of glycolic acid in the feed) - (Moles of glycolid acid in the discharge)] ÷ (Moles of glycolic acid in the feed) × 100%. Conversion rate of methoxyacetic acid = [(Moles of methoxyacetic acid in the feed) - (Moles of methoxyacetic acid in the discharge)] ÷ (Moles of methoxyacetic acid in the feed) × 100%.

### Example 1

300 g of acidic H-β molecular sieves (SiO₂/Al₂O₃=150) were loaded into a fixed bed reactor with an inner diameter of ϕ36 mm and a ϕ6 mm thermocouple sleeve inside the reactor. The carbonylation reaction between methylal and carbon monoxide occurs through the catalyst bed, and the product passes through a backpressure valve and enters the gas chromatography for online analysis after vaporization. The reaction conditions were as follows: the reaction temperature= 68°C, the reaction pressure= 6 MPa, the weight hourly space velocity of the methylal is 0.7 h⁻¹, and the molar ratio of carbon monoxide to methylal is 10:1. The reaction results are shown in Table 1 after 5 days of operation.

### Example 2

300 g of acidic H-Y molecular sieves (SiO₂/Al₂O₃=25) were loaded into a fixed bed reactor with an inner diameter of ϕ36 mm and a ϕ6 mm thermocouple sleeve inside the reactor. The carbonylation reaction between methylal and carbon monoxide occurs through the catalyst bed, and the product passes through a backpressure valve and enters the gas chromatography for on-line analysis after vaporization. The reaction conditions were as follows: the reaction temperature= 90°C, the reaction pressure= 5 MPa, the weight hourly space velocity of the methylal is 1.0 h⁻¹, and the molar ratio of carbon monoxide to methylal is 7: 1. The reaction results are shown in Table 1 after 5 days of operation.

### Example 3

300 g of acidic H-ZSM-5 molecular sieves (SiO₂/Al₂O₃=180) were loaded into a fixed bed reactor with an inner diameter of ϕ36 mm and a ϕ6 mm thermocouple sleeve inside the reactor. The carbonylation reaction between methylal and carbon monoxide occurs through the catalyst bed, and the product passes through a backpressure valve and enters the gas chromatography for on-line analysis after vaporization. The reaction conditions were as follows: the reaction temperature= 80°C, the reaction pressure= 8 MPa, the weight hourly space velocity of the methylal is 1.2 h⁻¹, and the molar ratio of carbon monoxide to methylal is 5:1. The reaction results are shown in Table 1 after 5 days of operation.

**Table 1. Results of methylal carbonylation reaction in Examples 1-3**

| Examples | Methylal conversion rate (%) | Methyl methoxyacetate selectivity (%) | Dimethyl ether selectivity (%) | Methyl formate selectivity (%) |
|---|---|---|---|---|
| 1 | 64.2 | 86.9 | 7.8 | 3.6 |
| 2 | 49.8 | 88.8 | 6.8 | 3.2 |
| 3 | 40.4 | 80.2 | 12.0 | 5.5 |

In the present application, the methylal and carbon monoxide that are not converted in Examples 1 to 3 can be recycled to the carbonylation reactor for the carbonylation reaction as shown in FIG. 1 to make full use of the raw material and to improve the utilization rate of the raw material.

### Example 4

300 g of acidic H-ZSM-5 molecular sieve (SiO₂/Al₂O₃=30) catalyst was loaded into a fixed bed reactor with an inner diameter of ϕ36 mm and a ϕ6 mm thermocouple sleeve inside the reactor. Methyl methoxyacetate prepared in Examples 1-3 was separated and hydrolyzed with water through the catalyst bed. The products were collected by condensation, weighed, and analyzed by gas chromatography and liquid chromatography, and the non-condensable gases were analyzed online by gas chromatography. The reaction conditions were as follows: the reaction temperature = 180°C, the reaction pressure = 0.1 MPa, the weight hourly space velocity of methyl methoxyacetate = 1.5 h⁻¹, the molar ratio of water to methyl methoxyacetate = 2: 1, and the flow rate of the carrier gas hydrogen = 1.5 L/min. The reaction results are shown in Table 2 after 5 days of operation.

### Example 5

300 g of acidic H-ZSM-35 molecular sieve (SiO₂/Al₂O₃=20) catalyst was loaded into a fixed bed reactor with an inner diameter of ϕ36 mm and a ϕ6 mm thermocouple sleeve inside the reactor. Methyl methoxyacetate prepared in Examples 1-3 was separated and hydrolyzed with water through the catalyst bed. The products were collected by condensation, weighed, and analyzed by gas chromatography and liquid chromatography, and the non-condensable gases were analyzed online by gas chromatography. The reaction conditions were as follows: the reaction temperature = 170°C, the reaction pressure = 0.12 MPa, the weight hourly space velocity of methyl methoxyacetate = 0.5 h⁻¹, the molar ratio of water to methyl methoxyacetate = 1: 1, and the flow rate of the carrier gas hydrogen = 1.5 L/min. The reaction results are shown in Table 2 after 5 days of operation.

### Example 6

300 g of acidic H-MCM-22 molecular sieve (SiO₂/Al₂O₃=40) catalyst was loaded into a fixed bed reactor with an inner diameter of ϕ36 mm and a ϕ6 mm thermocouple sleeve inside the reactor. Methyl methoxyacetate prepared in Examples 1-3 was separated and hydrolyzed with water through the catalyst bed. The products were collected by condensation, weighed, and analyzed by gas chromatography and liquid chromatography, and the non-condensable gases were analyzed online by gas chromatography. The reaction conditions were as follows: the reaction temperature = 190°C, the reaction pressure = 0.2 MPa, the weight hourly space velocity of methyl methoxyacetate = 0.4 h⁻¹, the molar ratio of water to methyl methoxyacetate = 3:1, and the flow rate of the carrier gas hydrogen = 1.5 L/min. The reaction results are shown in Table 2 after 5 days of operation.

**Table 2. Results of methyl methoxyacetate hydrolysis reaction in Examples 4-6**

| Examples | Methyl methoxyacetate conversion rate (%) | Methyl glycolate selectivity (%) | Methoxyacetic acid selectivity (%) | Glycolic acid selectivity (%) | Methanol selectivity (%) | Dimethyl ether selectivity (%) |
|---|---|---|---|---|---|---|
| 4 | 47.4 | 44.0 | 14.9 | 4.9 | 17.6 | 15.8 |
| 5 | 56.6 | 45.8 | 12.7 | 3.8 | 15.9 | 18.5 |
| 6 | 50.3 | 43.9 | 13.0 | 3.9 | 16.6 | 19.8 |

In the present application, the methyl methoxyacetate that is not converted in Examples 4 to 6 can be recycled to the hydrolysis reactor for hydrolysis reaction as shown in FIG. 1 to make full use of the raw material and to improve the utilization rate of the raw material.

### Example 7

300 g of quartz sand particles with a grain size of 3 mm were loaded into a fixed bed reactor with an inner diameter of ϕ36 mm and a ϕ6 mm thermocouple sleeve inside the reactor. The methoxyacetic acid, glycolic acid, methanol, and dimethyl ether prepared in Examples 4 to 6 were separated and passed through the quartz sand bed to undergo an esterification reaction. The products were collected by condensation, weighed, and analyzed by gas chromatography and liquid chromatography, and the non-condensable gases were analyzed online by gas chromatography. The reaction conditions were as follows: the reaction temperature = 180°C, the reaction pressure = 0.1 MPa, the flow rate of methoxyacetic acid = 120 g/h, and methoxyacetic acid: glycolic acid: methanol: dimethyl ether (molar ratio) = 5:1:10:10. The reaction results are shown in Table 3 after 5 days of operation.

### Example 8

300 g of silicon oxide particles with a grain size of 3 mm were loaded into a fixed bed reactor with an inner diameter of ϕ36 mm and a ϕ6 mm thermocouple sleeve inside the reactor. The methoxyacetic acid, glycolic acid, and dimethyl ether prepared in Examples 4 to 6 were separated and passed through the silicon oxide bed to undergo an esterification reaction. The products were collected by condensation, weighed, and analyzed by gas chromatography and liquid chromatography, and the non-condensable gases were analyzed online by gas chromatography. The reaction conditions were as follows: the reaction temperature = 200°C, the reaction pressure = 0.5 MPa, the flow rate of methoxyacetic acid = 120 g/h, and methoxyacetic acid: glycolic acid: dimethyl ether (molar ratio) = 6:1:7. The reaction results are shown in Table 3 after 5 days of operation.

### Example 9

300 g of glass particles with a grain size of 3 mm were loaded into a fixed bed reactor with an inner diameter of ϕ36 mm and a ϕ6 mm thermocouple sleeve inside the reactor. The methoxyacetic acid, glycolic acid, and dimethyl ether prepared in Examples 4 to 6 were separated and passed through the glass bed to undergo an esterification reaction. The products were collected by condensation, weighed, and analyzed by gas chromatography and liquid chromatography, and the non-condensable gases were analyzed online by gas chromatography. The reaction conditions were as follows: the reaction temperature = 300°C, the reaction pressure = 0.2 MPa, the flow rate of methoxyacetic acid = 120 g/h, and methoxyacetic acid: glycolic acid: dimethyl ether (molar ratio) = 5:1:8. The reaction results are shown in Table 3 after 5 days of operation.

### Example 10

The raw materials of methoxyacetic acid, glycolic acid and methanol were loaded into a 1 L kettle reactor with reaction temperature = 40°C, reaction pressure = 0.1 MPa, mass of methoxyacetic acid = 50 g, methoxyacetic acid: glycolic acid: methanol (molar ratio) = 5:1:10, and magneton stirring. The reaction results are shown in Table 3 after running for 4 hours.

### Example 11

The raw material of methoxyacetic acid, glycolic acid and methanol prepared in Examples 4 to 6 were separated and loaded into a 1 L kettle reactor with reaction temperature = 90°C, reaction pressure = 0.1 MPa, mass of methoxyacetic acid = 50 g, D001 strong-acid cation exchange resin (Dandong Mingzhu Co.) = 5 g, methoxyacetic acid: glycolic acid: methanol (molar ratio) = 5:1:10, and magnetron stirring. The reaction results are shown in Table 1 after running for 4 hours.

**Table3. Results of esterification reaction in Examples 7-11**

| Examples | Conversion rate of methoxyacetic acid (%) | Conversion rate of glycolic acid (%) |
|---|---|---|
| 7 | 75.5 | 99.5 |
| 8 | 80.4 | 99.1 |
| 9 | 94.8 | 99.6 |
| 10 | 35.8 | 80.2 |
| 11 | 82.2 | 96.7 |

In the present application, the methyl methoxyacetate obtained in Examples 7 to 11 can be recycled to the hydrolysis reactor for hydrolysis reaction to make full use of the product and to improve the atomic economy.

The above embodiments are merely some of the embodiments of the present application, and do not limit the present application in any form. Although the present application is disclosed above with the preferred embodiments, the present application is not limited thereto. Some changes or modifications made by any technical personnel familiar with the profession using the technical content disclosed above without departing from the scope of the technical solutions of the present application are equivalent to equivalent implementation cases and fall within the scope of the technical solutions.

## Claims

1. A method for preparing a methyl ester compound, wherein a raw material containing a carboxyl compound, methanol and/or dimethyl ether is passed through a reactor and an esterification reaction occurs to obtain the methyl ester compound;
wherein the carboxyl compound is at least one selected from the group consisting of the compounds shown in Formula I;
R-CH₂-COOH Formula I
R is methoxy or hydroxy;
the methyl ester compound comprises at least one of methyl methoxyacetate, methyl glycolate.

2. The method according to claim 1, wherein conditions of the esterification reaction are as follows: a reaction temperature ranges from 40°C to 300°C and a reaction pressure ranges from 0.1 MPa to 0.5 MPa;
the esterification reaction is carried out without a catalyst or under a condition of an esterification catalyst;
the esterification catalyst is a solid catalyst insoluble in the raw material and the product.

3. The method according to claim 2, wherein the solid catalyst is at least one of an acidic molecular sieve, an acidic cation exchange resin.

4. The method according to claim 1, wherein the esterification reaction is carried out on at least one inert composition selected from the group consisting of quartz sand, alumina, silicon oxide, silicon carbide, glass, ceramics.

5. The method according to claim 1, wherein the raw material is methoxyacetic acid, methanol and/or dimethyl ether; or
the raw material is glycolic acid, methanol and/or dimethyl ether; or
the raw material is methoxyacetic acid, glycolic acid, and methanol; or
the raw material is methoxyacetic acid, glycolic acid, and dimethyl ether; or
the raw material is methoxyacetic acid, glycolic acid, methanol, and dimethyl ether.

6. The method according to claim 1, wherein the raw material contains methoxyacetic acid, glycolic acid, methanol, and dimethyl ether; the molar ratio of methoxyacetic acid/glycolic acid in the raw material is from 3:1 to 10:1, and the molar ratio of (methanol + dimethyl ether)/ (methoxyacetic acid + glycolic acid) is from 1:1 to 5:1.

7. The method according to claim 1, wherein the raw material does not contain methanol, the molar ratio of methoxyacetic acid/ glycolic acid is from 3:1 to 10:1, and the molar ratio of dimethyl ether/ (methoxyacetic acid + glycolic acid) is from 1:1 to 5:1.

8. The method according to claim 1, wherein the raw material does not contain methanol and glycolic acid, and the molar ratio of dimethyl ether/methoxyacetic acid is from 1:1 to 5:1.

9. The method according to claim 1, wherein the raw material does not contain dimethyl ether; the molar ratio of methoxyacetic acid/glycolic acid is from 3:1 to 10:1, and the molar ratio of methanol/ (methoxyacetic acid + glycolic acid) is from 1:1 to 5:1.

10. The method according to claims 1 or 5, wherein the raw material is generated through a hydrolysis reaction of methyl methoxyacetate.

11. The method according to claim 5, wherein the raw material containing methoxyacetic acid, glycolic acid, methanol, and dimethyl ether is generated through a hydrolysis reaction of methyl methoxyacetate.

12. The method according to claim 1, wherein the reactor is one of a fixed bed reactor or a kettle reactor.

13. The method according to claim 1, wherein conditions of the reaction comprise a carrier gas selected from one of nitrogen, argon, helium, hydrogen, carbon monoxide, carbon dioxide in any amount.

14. A method for preparing a methyl glycolate, wherein the method comprises the following steps:
a) passing a raw material containing methylal and carbon monoxide through a reactor carrying an acidic molecular sieve catalyst, and performing a carbonylation reaction under a predetermined condition to generate a product containing methyl methoxyacetate, dimethyl ether, and methyl formate, which is separated to obtain a methyl methoxyacetate;
b) passing a raw material containing the methyl methoxyacetate obtained in step a) and water through a reactor carrying an acidic molecular sieve catalyst, and performing a hydrolysis reaction under a predetermined condition to generate a product containing methyl glycolate, glycolic acid, methoxyacetic acid, methanol, and dimethyl ether, which is separated to obtain the methyl glycolate; and
c) passing the glycolic acid, the methoxyacetic acid, the methanol and dimethyl ether obtained in step b) through a reactor, and performing an esterification reaction under a predetermined condition to generate a product containing methyl methoxyacetate and methyl glycolate, which is separated to obtain the methyl glycolate.

15. The method according to claim 14, wherein the method further comprises step a'): returning the unreacted methylal and carbon monoxide in step a) to the reactor of step a) to continue the carbonylation reaction.

16. The method according to claim 14, wherein the method further comprises step b'): returning the unreacted methyl methoxyacetate in step b) to the reactor of step b) to continue the hydrolysis reaction with water.

17. The method according to claim 14, wherein the method further comprises step c'): passing the product methyl methoxyacetate obtained in step c) into the reactor of step b) for the hydrolysis reaction with water.

18. The method according to claim 14, wherein the acidic molecular sieve catalyst in step a) or step b) is at least one selected from the group consisting of an acidic molecular sieve with MFI structure, an acidic molecular sieve with Y structure, an acidic molecular sieve with FER structure, an acidic molecular sieve with BEA structure, an acidic molecular sieve with MOR structure, an acidic molecular sieve with MWW structure.

19. The method according to claim 14, wherein the acidic molecular sieve catalyst in step a) or step b) is at least one of a hydrogen-type ZSM-5 molecular sieve, a hydrogen-type Y molecular sieve, a hydrogen-type ZSM-35 molecular sieve, a hydrogen-type β molecular sieve, a hydrogen-type mordenite molecular sieve, and a hydrogen-type MCM-22 molecular sieve.

20. The method according to claim 14, wherein step a) or step b) further comprises introducing a carrier gas into the reactor, wherein the carrier gas comprises at least one of nitrogen, argon, helium, hydrogen, carbon monoxide, or carbon dioxide in any amount.

21. The method according to claim 14, wherein reaction conditions in step a) are: a reaction temperature ranges from 60°C to 140°C, a reaction pressure ranges from 2 MPa to 10 MPa, a weight hourly space velocity of the methylal ranges from 0.2 h⁻¹ to 10.0 h⁻¹, and a molar ratio of the carbon monoxide to the methylal ranges from 2:1 to 20:1.

22. The method according to claim 14, wherein reaction conditions in step b) are: a reaction temperature ranges from 140°C to 220°C, a reaction pressure ranges from 0.1 MPa to 0.5 MPa, a weight hourly space velocity of the methyl methoxyacetate ranges from 0.1 h⁻¹ to 3.0 h⁻¹, and a molar ratio of the water to the methyl methoxyacetate ranges from 0.5:1 to 8:1.

23. The method according to claim 14, wherein reaction conditions in step c) are: a reaction temperature ranges from 40°C to 300°C, a reaction pressure ranges from 0.1 MPa to 0.5 MPa, and a molar ratio of (methanol + dimethyl ether)/ (glycolic acid + methoxyacetic acid) ranges from 0.5:1 to 8:1.

24. The method according to claim 14, wherein a reaction state in step a) is a gas-liquid-solid three-phase reaction state.

25. The method according to claim 14, wherein the esterification reaction in step c) is carried out without a catalyst or under an esterification catalyst condition.

26. The method according to claim 25, wherein the esterification catalyst is a solid catalyst insoluble in the raw material and the product.

27. The method according to claim 26, wherein the solid catalyst is at least one of an acidic molecular sieve, an acidic cation exchange resin.

28. The method according to claim 25, wherein the esterification reaction is carried out on at least one inert composition selected from the group consisting of quartz sand, alumina, silicon oxide, silicon carbide, glass, ceramics.
